# EUROPEAN PATENT APPLICATION

(11) **EP 0 901 017 A2**
(43) Date of publication of application: **10.03.1999**
(21) Application number: 98116260.5
(22) Date of filing: 28.08.1998
(51) Int. Cl.: G01N 33/38, G01N 11/00, B60P 3/16

(54) **Method of checking the slump of a ready-mix concrete load**

(30) Priority: 02.09.1997 GB 9718829
(71) Applicant: Tarmac Heavy Building Materials UK Limited, Ettingshall, Wolverhampton WV4 6JP (GB)
(72) Inventor: Turner, Alexandre Houghton, Cannock, WS11 1HY (GB); Taylor, Anthony Robert, Longden-on-Tern, Telford, TF6 6LH (GB); Humpish, Nick, Priorslee, Telford, TF2 9SX (GB)
(74) Representative: Lucking, David John

(57) **Abstract**

A method is described of checking the slump value of a ready-mix concrete load at the point of delivery in a delivery vehicle (10) of the kind having a rotatable drum (11) in which the concrete load is contained. The method comprises determining a measure of the work required to rotate the drum (11) to produce first data, transmitting information to a base (16), the information dependant upon the first data, determining the slump value of the concrete load from the first data either at the point of delivery or from the transmitted information at the base (16) and storing the information or the slump value at the base (16) whereby an independent record of the slump value of the concrete load at the point of delivery is made at the base (16).

## Description

### Description of Invention

This invention relates to a method of checking the slump of a ready-mix concrete load.

Large volumes of concrete mix for use on building sites etc., are generally provided by mixer-trucks that transport the manufactured concrete mix from the manufacturing unit to the building site. Mixer trucks convey the concrete in a mixer carried on the rear of the truck which rotates continuously during transport to ensure that the concrete maintains a uniform mix composition. At delivery the concrete mix is emptied from the mixer e.g. via a chute, to a desired location.

The compositions of concrete mixes vary depending upon the particular end use for which the concrete is required and may comprise varying amounts of sand, cement, aggregate and water and other additives. Specific concrete mixes are normally identified by the workability of the mix which is a measure of the flow and compacting factors of a specific mix. Workability is measured by what is known as the "slump" value of the mix. The slump value is conventionally determined by loading a given volume of mix into a conical gauge and measuring the value (depth) of liquid that separates out on the surface after a given period of time. This measurement has to be performed by skilled personnel.

The "slump" value of a specific concrete mix required for a particular use is normally specified by an architect/engineer so that the concrete achieves its design performance criteria, for example, meets the criteria for the foundations of a building such as an office block.

The slump value of a specific concrete mix may vary in the time period between the preparation of the original mix and delivery to a remote site through loss of water which may occur as a result of high ambient temperature and/or unavoidable delays in delivery, e.g. as a result of road congestion and is prone to unauthorised adjustment by delivery vehicle drivers and building site workers. It is therefore desirable to have a convenient means of independently checking the slump value of a specific concrete mix at the point of delivery at least and maintaining an independent record of the slump value of the concrete load as delivered..

The value of such information is first to ensure that the mix delivered has the correct slump value and secondly to have a record of the slump value at the time of delivery in case of subsequent failure of the concrete, to facilitate combating any allegation that the failure resulted from the delivery of a mix with an incorrect slump value.

It is known to measure the slump of concrete of a load in a drum of a delivery vehicle utilising an autonomous sensing system provided on the delivery vehicle but no independent record can be made of the specification of the concrete load which is delivered.

According to one aspect of the invention we provide a method of checking the slump value of a ready-mix concrete load at the point of delivery in a delivery vehicle of the kind having a rotatable drum in which the concrete load is contained the method comprising determining a measure of the work required to rotate the drum to produce first data, transmitting information to a base, the information dependant upon the first data, determining the slump value of the concrete load from the first data either at the point of delivery or from the transmitted information at the base and storing the information or the slump value at the base whereby an independent record of the slump value of the concrete load at the point of delivery is made at the base.

Thus a permanent independent record can be made at the base as confirmation of the specification of the concrete load delivered.

In a typical ready-mix concrete delivery vehicle, the drum is rotated by a hydraulic motor. In this case, to obtain a measure of the work required to rotate the drum, means are provided to sense the speed of rotation of the drum and the pressure exerted on the hydraulic system rotating the drum by the concrete mix. The speed and pressure may thus be correlated with known slump values for that particular mix.

Alternatively where the drum is rotated by other means such as an electric or other motor, the work required to rotate the drum at a predetermined speed of rotation may be measured by other means. Another alternative would be to sense fuel consumption of the engine of the delivery vehicle which provides power to rotate the drum.

In each case means may be provided to sense the speed of the drum and to use the sensed speed to produce the first data, but alternatively the method may be performed with the drum set to rotate at a predetermined speed.

If desired, means such as for example load cells may be provided to sense the mass of the load and to use the sensed mass in determining the slump value which is useful where the concrete load is a partial load relative to the capacity of the drum.

Where the slump value of the concrete load is determined at the base, the transmitted information may comprise the first data or processed first data, but preferably the slump value of the concrete load is determined at the point of delivery and if desired means may be provided on the vehicle to display the slump value of the load.

In one arrangement the first data and/or the information for transmission is stored in an information storage means, such as a computer data carrier, or a magnetic tape or computer memory, which is provided on the vehicle, and a printing means may be provided so that the slump value may be printed out.

The information may thus be transmitted to base in one embodiment by downloading the information at the base e.g. into a computer data storage means, or at an intermediate station such as the vehicle garage, when the vehicle returns to base or travels to the intermediate station.

In another embodiment the information may be transmitted by a radio and/or telegraphic link between the vehicle and the base, and such transmission may or may not be simultaneous with the determing of the slump value.

The method may include comparing the slump value with reference data at the base, and transmitting a second signal from the base to the delivery vehicle which second signal indicates whether or not the slump value of the concrete load is still within a predetermined range.

The second signal where provided, may comprise an instruction to personnel at the point of delivery, such as an instruction not to deliver the concrete load or to carry out remedial steps such as adding water to "adjust" the slump of the load, and/or may comprise a readout of the slump value of the load.

The information howsoever transmitted to base may contain information concerning the identity of the delivery vehicle and/or the driver of the vehicle and/or the load and/or the location of the point of delivery, the time of delivery etc.

Preferably at least at the base, the method steps are automated e.g. under computer control, so that a record of the slump of the concrete load being delivered can be made automatically as a data record or print-out for examples.

The method may include monitoring the addition of any water to the concrete load subsequent to dispatch of the delivery vehicle, from base e.g. utilising a flow sensor in a water feed from a water tank of the vehicle to the drum, to produce second data, the transmitted information information including information dependant on the second data.

The method may comprise determining the slump value of the concrete load at base prior to dispatch of the delivery vehicle by determining a measure of the work required to rotate the drum to produce data and transmitting information dependant upon the data

According to a second aspect of the invention we provide an apparatus for performing the method of any one of the preceding claims comprising a delivery vehicle for delivering a ready-mix concrete load to a delivery site, means to rotate a drum of the delivery vehicle which contains the concrete load, means to determine a measure of the work required to rotate the drum to produce first data related thereto, means to transmit information which is dependant on the first data, means at a base to receive the information, means to determine the slump value of the concrete load from the first data at the point of delivery or from the transmitted information at the base.

The apparatus may include means at the base to compare the information relevant to the slump value with reference data and means at the base to transmit a second signal which indicates whether or not the slump value of the concrete load is within a predetermined range, and means at the point of delivery to receive the second signal.

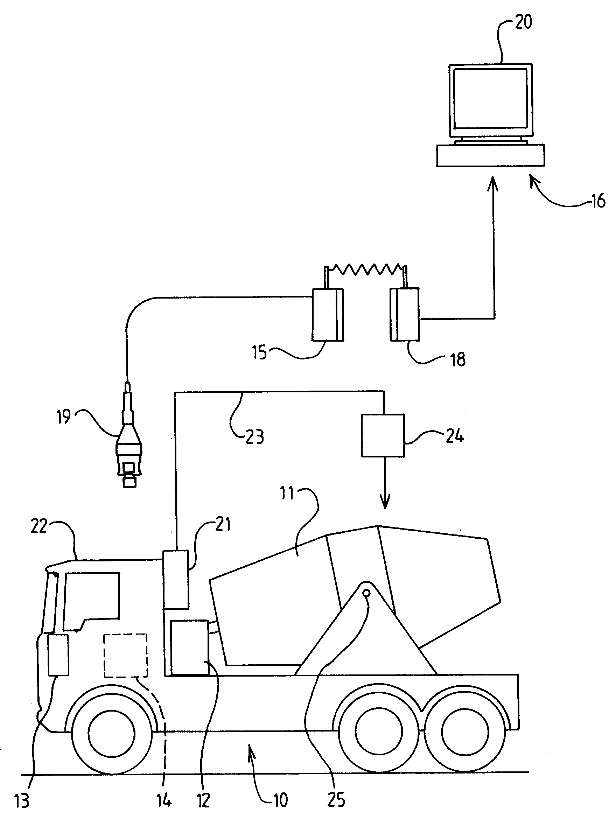

An embodiment of the invention will now described with reference to the accompanying drawing which is an illustration of an apparatus which may be utilised to perform the method of the invention.

Referring to the drawing there is shown a delivery vehicle 10 of the kind having a drum 11 which may be rotated by any desired drum drive means, such as in this example a hydraulic motor 12. In use the drum 11 contains a load of ready-mix concrete which is produced at a concrete manufacturing site and contains water, sand, aggregate and cement to a predetermined specification including slump value, for a particular application. As the drum 11 rotates, the uniformity of the mix is preserved. Usually the slump of the mix would be determined at the manufacturing site before the delivery vehicle 10 is dispatched to a point of delivery, such as a building site etc., by a conventional slump measuring method, although the slump value could be determined by the method described hereinafter.

The drum 11 may in transit be rotated by the motor 12 to a speed of only a few revolutions per minute but during dermination of the slump value by the method of the invention, may typically be rotated at a speed of 9-10 revolutions per minute only.

In accordance with the present invention, the delivery vehicle 10 comprises a transceiver 15 for transmitting and receiving e.g. short wave signals to enable communication between the vehicle 10 and a base 16 which may or may not be at the concrete manufacturing site. At the base 16 there is a further transceiver 18.

In carrying out the method of the invention, the hydraulic pressure required to rotate a mixing drum at a substantially constant speed of rotation, say 9-10 rpm, is measured by all appropriate pressure sensing means 19 to produce first data. This pressure will be indicative of the work required to rotate the drum 11 at the predetermined speed of rotation which in turn will depend on the slump value of the load under delivery. For a controlled drum speed, the slump value and pressure show a negative linear relationship. Thus, the first data, which may be a simple signal containing the hydraulic pressure measurements, will depend on the slump value of the concrete load.

Information which is dependant upon the first data, is then transmitted by the transceiver 15 to the base 16. The information may include other information such as an identification of the delivery vehicle 10 or of the operator delivering the load, and/or of the speed of rotation of the drum 11 and/or the time/date etc, or even the location of the point of delivery.

The transceiver 18 at the base may be set to "listen out" for transmitted signals from the concrete delivery vehicle 10. On receipt of die transmitted information at the base 16, a computer control means 20 determines the slump value of the concrete load at the point of delivery. This may be achieved utilising comparative data obtained previously by way of calibration of the system. For example slump values for a range of first data may be recorded in a look-up table and the computer control means 20 may determine from comparison and calculation a corresponding slump value for any particular first signal.

Next, the slump value may be compared with reference data stored at the base 16 concerning the specification of the concrete load which ought to be delivered at the point of delivery. In the event that any discrepancy is found between the slump value of the concrete load at the delivery site and the slump value of the concrete which was ordered at the site such that the slump value of the concrete load being delivered is determined to be outside of an acceptable tolerance range, the control means 20 may either alert an operator at the base 16 to send an appropriate second signal to the personnel at the delivery site, or else automatically send a signal to the delivery site via transceiver 18.

Such signal may simply be a warning to the personnel at the point of delivery that there is such discrepancy, e.g. by displaying to the operator on a display 13 provided on the vehicle 10, the slump value of the concrete load, and/or some instruction such as an instruction not to deliver the load, or to add water or other additive to the load to "adjust" the slump value. In this latter event, the slump value of the concrete load may be re-tested by performing the method of the invention again, or otherwise.

In each case, an independent data or printed record of the slump value of the concrete load, before and after and corrective action, may be made at the base 16 for reference purposes.

Various modifications may be made without departing from the scope of the invention.

For example, instead of a direct radio transmission link between the vehicle 10 and the base 16, telephonic communication may be used. Particularly the second signal from the base 16 to the point of delivery may be by telephonic link whilst the first signal may be sent by radio link as described, or vice versa.

Although preferably the transceiver 15 is mounted on and is a component part of the delivery vehicle, the transceiver 15 or other first signal transmission means may otherwise be provided at the point of delivery.

In another arrangement, instead of a transmission by radio or telephonic link, the information may be transmitted from the delivery vehicle 10 to the base 16 in another manner altogether. For example, the delivery vehicle 10 may comprise some computer data storage means 14, as shown in dotted lines, to store the first data and/or a measure of the slump value of the concrete as delivered. When the vehicle 10 returns to base or an intermediate station, that information may be down loaded to a computer control means 20 of the base 16 eg via an electronic interface direct link, or by some kind of removable data storage mechanism such as a computer data disc or tape. In each case, an independent record can thus be made at the base 20 of the slump value of the concrete as delivered.

Preferably a determination of information relevant to slump value from the first data, is made at the point of delivery of the concrete load. However, the operator at the point of delivery may obtain a reading of slump value of the load, and such reading may be printed out on a printing means provided on the delivery vehicle 10 or at least, the slump value as determined may be displayed on a display means 13 of the vehicle eg within an operator's cab 22 thereof.

In accordance with the present invention, the delivery vehicle 10 has a water tank 21 and a conduit 23 extends from the water tank 21 into the drum 11 of the vehicle 10 so that water may be added to the concrete load. Preferably, there is provided a flow sensor 24 to sense the volume of water which is fed from the tank 21 to the drum 11 subsequent to the delivery vehicle 10 being dispatched from the base 16 so that any unauthorised adjustment of the concrete load can be determined. The information which is transmitted from the delivery vehicle 10 to the base 16 may thus be dependent upon second data produced by the flow sensing means 24.

If desired, particularly where concrete loads are delivered by the delivery vehicle 10 of less than the capacity of the drum 11, means may be provided to sense the mass of the concrete load and to use the sensed mass in determining the slump value in the delivery vehicle 10 and/or at base 20.

Instead of measuring the work required to rotate the drum 11 by the method described above, any other suitable method may be employed. For example a measure of the work required to rotate the drum 11 at a particular speed of rotation is determined by sensing fuel consumption of an engine of the delivery vehicle 10 providing power to rotate the drum, in which case means would need to be provided to sense the speed of rotation of the drum 11 and to use the sensed speed in producing the first data.

The features disclosed in the foregoing description, or the following claims, or the accompanying drawings, expressed in their specific forms or in terms of a means for performing the disclosed function, or a method or process for attaining the disclosed result, as appropriate, may, separately or in any combination of such features, be utilised for realising the invention in diverse forms thereof.

## Claims

1. A method of checking the slump value of a ready-mix concrete load at the point of delivery in a delivery vehicle (10) of the kind having a rotatable drum (11) in which the concrete load is contained the method comprising determining a measure of the work required to rotate the drum (11) to produce first data, transmitting information to a base (16), the information being dependant upon the first data, determining the slump value of the concrete load from the first data either at the point of delivery or from the transmitted information at the base (16) and storing the information or the slump value at the base (16) whereby an independent record of the slump value of the concrete load at the point of delivery is made at the base (16).

2. A method according to claim 1 characterised in that the drum (11) is rotated by a hydraulic motor (12) and a measure of the work required to rotate the drum (11) at a particular speed of rotation is determined by sensing the pressure exerted on the hydraulic system rotating the drum (11), by the concrete mix and wherein the speed of rotation and hydraulic pressure are correlated with known slump values for that particular mix to determine the information relevant to the slump value of the concrete load.

3. A method according to claim 2 characterised in that the measure of work required to rotate the drum is determined at a predetermined drum rotational speed.

4. A method according to any one of the preceding claims characterised in that the slump value of the concrete load is determined at the base (16), and the information which is transmitted comprises the first data or processed first data.

5. A method according to any one of the preceding claims characterised in that the slump value of the concrete load is determined at the point of delivery and means (13) are provided on the vehicle (10) to display the slump value of the concrete load the first data and/or the information being stored in an information storage means (14) provided on the vehicle.

6. A method according to any one of the preceding claims wherein means (14) are provided on the delivery vehicle (10) for storing the first data and/or the information determined therefrom relevant to the slump value of the concrete load at the point of delivery and the method includes transmitting the information to base (16) by downloading the information at the base (16) or an intermediate station when the delivery vehicle (10) returns to base or travels to the intermediate station.

7. A method according to claim 6 characterised in that the information is downloaded via an electronic interface and/or from removable data storage means.

8. A method according to any one of claims 1 to 5 characterised in that the first data and/or the information determined therefrom relevant to the slump value of the concrete load at the point of delivery is transmitted by a radio and/or telegraphic link, the method including comparing the slump value with reference data at the base (16), transmitting a second signal from the base (16) to the delivery vehicle which second signal indicates whether or not the slump value of the concrete load is within a predetermined range.

9. A method according to any one of the preceding claims characterised in that the transmitted information contains information concerning the identity of the delivery vehicle (10) and/or the driver of the vehicle and/or the load and/or the location of the point of delivery.

10. A method according to any one of the preceding claims characterised in that the method includes monitoring any addition of water to the concrete load subsequent to dispatch of the delivery vehicle from base (16) to produce second data, the transmitted information including information dependant on the second data, the water monitoring being preferred by means of a water feed conduit (23) from a water tank of the delivery vehicle (10) to the drum (11), having thereon a flow sensor (24) which produces the second data dependent upon the amount of water which has flowed through the conduit (23).

11. A method according to any one of the preceding claims characterised in that the method comprises determining the slump value of the concrete load at base (16) prior to dispatch of the delivery vehicle by determining a measure of the work required to rotate the drum (11) to produce data and transmitting information dependant upon the data to a control means (20) of the base (16) and storing the slump value thus determined.

12. An apparatus for performing the method of any one of the preceding claims comprising a delivery vehicle (10) for delivering a ready-mix concrete load to a delivery site, means (12) to rotate a drum (11) of the delivery vehicle (10) which contains the concrete load, means (19) to determine a measure of the work required to rotate the drum (11) to produce first data related thereto, means (18) to transmit information which is dependant on the first data, means (18) at a base (16) to receive the information means (20) to determine the slump value of the concrete load from the first data at the point of delivery or from the transmitted information at the base (16).
